# EUROPEAN PATENT APPLICATION

(11) **EP 4 495 251 A1**
(43) Date of publication of application: **22.01.2025**
(21) Application number: 23186395.2
(22) Date of filing: 19.07.2023
(51) Int. Cl.: C12Q 1/6844, C12Q 1/6869

(54) **METHOD FOR SEQUENCING WITHIN A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A method for sequencing within a biological sample is provided comprising the following steps: providing the biological sample comprising at least one target sequence; amplifying within the biological sample the target sequence with an isothermal amplification method in order to generate at least one amplified target sequence; sequencing within the biological sample the amplified target sequence.

## Description

### Technical field

The invention relates to a method for amplification and sequencing of a target sequence within the biological sample.

### Background

The determination of proteomic, genomic and transcriptomic data from biological samples remains a focus in the life sciences. Generally optical readouts are used to identify target structures that are marked with fluorescent markers and that specifically attach to target structures. This enables the identification of proteins, for example. In addition, by using ISH-probes, short nucleic acid sequences may be identified similarly. In combination with imaging the biological sample, the light emitted by the markers or ISH-probes at a particular location in the biological samples may be used to assign the identified proteins or short nucleic acids sequences a location within the biological sample. However, it remains difficult to sequence nucleic acids within biological samples without requiring destruction of the biological sample and extraction of target sequences prior to sequencing.

### Summary

It is an object to provide a method for efficiently determining the sequence of a target sequence within a biological sample

The aforementioned object is achieved by the subject-matter of the independent claims. Advantageous embodiments are defined in the dependent claims and the following description.

A method for sequencing within a biological sample is provided comprising the following steps: providing the biological sample comprising at least one target sequence; amplifying within the biological sample the target sequence with an isothermal amplification method in order to generate at least one amplified target sequence; sequencing within the biological sample the amplified target sequence.

For example, the biological sample may be a tissue section, such as from a biopsy. The target sequence is preferably a nucleic acid strand of the biological sample, such as a mRNA or a DNA. For example, in case the biological sample is a tissue section, the cells of the tissue section comprise nucleic acid sequences, that may be sequenced. The amplification and the sequencing steps are carried out within the biological sample. Carrying out these steps *in situ* enables generating spatially resolved sequencing data, i.e. sequence reads (e.g. AAATGCCAA) that are located in in 2D (e.g. single plane acquisition of thin samples like cell cultures) or 3D samples (e.g. organoids, cleared samples).

Preferably, a biological sample in the sense of the present invention may be: a cell culture sample, 3D cell culture, organoid, spheroid, tissuoid, tissue section, liquid biopsy, a lysate, a plasma proteome sample, any bodily fluid sample, a synthetic biology sample like engineered cells, tissues, organs, model organisms. To determine a sequence within or in situ of a biological sample refers to either an endogenous RNA or DNA target sequence, like mRNA or genetic loci, or to exogenously introduced nucleic acid targets, like for example particle, ISH probes, or other affinity reagents including but not limited to antibodies, nanobodies, and aptamers, which might be barcoded with a nucleic acid label or oligonucleotide barcode. In both cases the method achieves fast, robust, and easy amplification of nucleic acid target sequences and provides sequence data for the target nucleic acids, which may be used for low to ultra-high plex detection, i.e. identification of the target, and/or for the actual sequence analysis of such targets, including but not limited to the transcriptome-wide analysis of splicing, insertions, deletions, single-nucleotide polymorphisms (SNPs), and premature stops. The present invention further enables the method to be used to perform plasma proteomics, spatial proteomics, as well as quality control and therapy monitoring in cell therapy.

The isothermal amplification method is preferably carried out at an essentially constant temperature or in a narrow temperature range, requiring no thermocycling. These temperatures are considerably lower than for polymerase chain reaction amplification methods that are typically carried out in wide temperature ranges with temperatures up to 98 °C during denaturation steps.

Preferably, the isothermal amplification method comprises introducing a set of primers, in particular, comprising 4 to 6 primers, into the biological sample, the primers of the set of primers are configured to hybridise to the target sequence. This enables a specific initiation of amplification of the target sequence within the biological sample.

Preferably, the primers of the set of primers are configured to generate hairpin loops at ends of the target sequence, in particular, at either 5' or 3' ends of the target sequence. In particular, the primers of the set of primers cause the target sequence to loop back on itself in order to generate the hairpin loops. This enables efficiently generating free ends to initiate amplification.

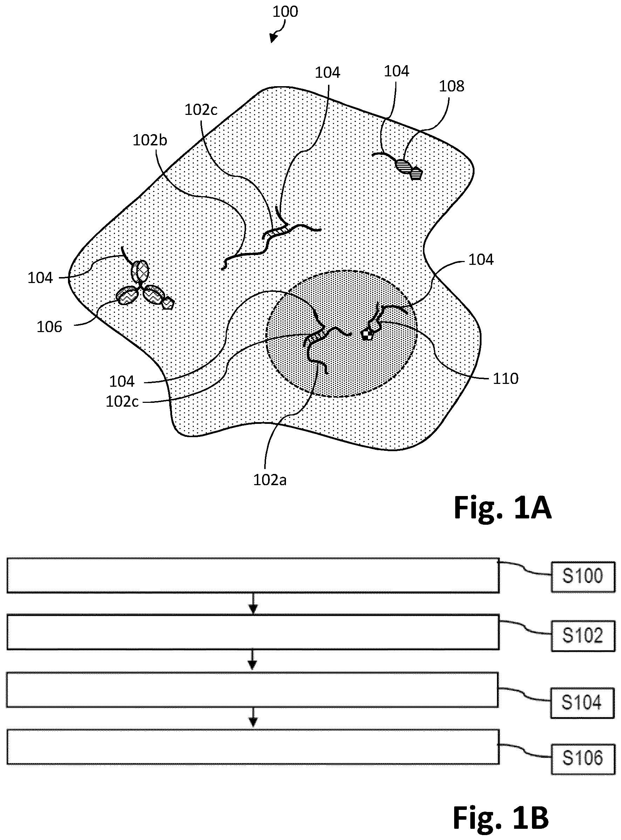

A specific example to generate hairpin loops with a set of primers is given by Notomi et al. 2000 (Notomi T, Okayama H, Masubuchi H, Yonekawa T, Watanabe K, Amino N, Hase T. Loop-mediated isothermal amplification of DNA. Nucleic Acids Res. 2000 Jun 15;28(12):E63 PMID: 10871386) and Podushkina et al., 2019 (Utilizing multiplex fluor LAMPs to illuminate multiple gene expressions in situ), in particular, with reference to Fig. 1 in Podushkina et al., 2019. Generally, loop-mediated isothermal amplification (LAMP) may be utilised for amplification of the target sequence. In case the target sequence is a mRNA, reverse transcription loop-mediated isothermal amplification (RT-LAMP) may be utilised.

Preferably, the set of primers comprises at least a first primer with a first region of the first primer being configured to hybridise to at least the target sequence towards a 3' end of the target sequence, and a second region of the first primer being configured to hybridise to the amplified target sequence downstream of the first region of the first primer. In particular, the first region of the first primer is at a 3' end of the first primer and the second region is at a 5' end. This enables efficient formation of a hairpin loop at a 3' end of the target sequence.

Preferably, the set of primers comprises at least a second primer with a first region of the second primer being configured to hybridise to the amplified target sequence towards a 3' end of the amplified target sequence, and a second region of the second primer being configured to hybridise to the amplified target sequence downstream of the first region of the second primer. In particular, the first region of the second primer is at a 3' end of the second primer and the second region is at a 5' end. This enables efficient formation of a hairpin loop at a 5' end of the target sequence.

Preferably, the amplified target sequence is a continuous sequence comprising multiple copies of the target sequence. Sequencing of the copies of the target sequence in the continuous sequence may be initiated by providing respective forward and reverse primers.

Preferably, the isothermal amplification method comprises introducing a strand displacing polymerase into the biological sample. This enables efficient generation of the amplified target sequence.

Preferably, the strand displaying polymerase is a Phi29 polymerase, a Bacillus subtilis DNA polymerase I, a T7 DNA polymerase, or a T4 DNA polymerase. This enables amplification at low temperature. For example, Phi29 polymerase works at 30°C and is particularly preferred because of low background generation in the biological sample at this temperature; Bacillus subtilis DNA polymerase I works at 37°C - 42°C; T4 and T7 DNA polymerase work at around 37°C.

Preferably, the biological sample is permeabilised and/or expanded, preferably prior to the step of amplifying the target sequence. The permeabilisation and/or the expansion of the biological sample may be carried out such that the structure of the biological sample essentially remains unchanged. This allows introduction of amplification and/or sequencing reagents into the biological sample. Further, expansion of the biological sample may improve generating spatially resolved sequencing data.

Preferably, the step of amplifying the target sequence is carried out at a substantially constant temperature in a range from 20°C to 75 °C, more preferably in a range from 30°C to 65°C, more preferably in a range from 30 °C to 50 °C, even more preferably in a range from 35 °C to 45 °C. This enables carrying out the amplification step without substantial damage to the biological sample due to heat. In particular, the temperature of the amplification step is the temperature the biological sample is at. Preferably, the temperature is held constant at a certain temperature within the range. Preferably, the sequencing step is similarly carried out at a temperature at or below the temperature of the amplification step.

Preferably, the step of amplifying the target sequence is carried out in a range from 0.5 min to 60 min. This enables efficiently generating a sufficient number of copies of the target sequence. Preferably the time for carrying out the step is in a range from 1 min to 15 min. This yields an approximate 100- to 10000-fold increase in copy number of the target sequence. In particular, this time interval is started after introducing the first set of primers.

Preferably, after the step of amplifying the target sequence, the amplified target sequence is crosslinked. This results in the amplified target sequence remaining in place within the biological sample. Further, this may improve generating spatially resolved sequencing data. For example, the amplified target sequence may be crosslinked to proteins of the biological sample, or a gel may be introduced into the biological sample. In addition or alternatively, at least one of the primers of the first set may comprise a chemical modification that enables its crosslinking.

Preferably, the biological sample comprises a second target sequence, and the step of amplifying the target sequence comprises introducing a second set of primers in order to amplify the second target sequence and the primers of the second set are specific to the second target sequence. This enables efficiently sequencing target sequences within the biological sample. Alternatively, the second set of primers may be introduced after amplification and sequencing of the target sequence.

Preferably, the step of sequencing the amplified target sequence comprises introducing labelled nucleotides into the biological sample. This enables efficient in situ sequencing and spatially assigning the generated sequencing data within the biological sample. The labels may comprise fluorophores, for example.

Preferably, the step of sequencing the amplified target sequence comprises detecting light emitted from the biological sample, in particular, when a nucleotide is incorporated into a complementary strand during replication of the amplified target sequence. This enables efficient *in situ* sequencing and spatially assigning the generated sequencing data within the biological sample. This may be based on sequencing-by-synthesis approaches such as: Illumina (Solexa) sequencing (reversible dye-terminator method and derived methods; originally described in Bentley et al. (2008) Accurate whole genome sequencing using reversible terminator chemistry. Nature 456, 53-59.; pyrosequencing originally described by Ronaghi et al. Real-time DNA sequencing using detection of pyrophosphate release. Analytical Biochemistry, 242(1), 84-89.; single molecule, real-time (SMRT) sequencing, which is commercialized by Pacific Biosciences and originally described in Eid et al. (2009) Real-time DNA sequencing from single polymerase molecules; or a derived or a related sequencing-by-synthesis approach. Such chemistries may be based on a single fluorescent dye (1-color), 2-colors, or 4-colors.

The step of sequencing the amplified target sequence preferably comprises detecting light emitted from the biological sample, in particularly, of the sequencing reaction or the labelled nucleotides, by means of an optical readout device. Such an optical readout device may be a microscope, in particular by a light microscope, by a light-field microscope, by a fluorescence microscope, by a confocal light microscope - in particular a STED (stimulated emission depletion) microscope - or by a super-resolution light microscope applying a localisation technology such as PALM (photo-activated localization microscopy), STORM (stochastic optical reconstruction microscopy), GSDIM (ground state depletion microscopy followed by individual molecule return), or a technology according to SIM (spatially modulated illumination). This further enables precise spatial assignment of the sequencing data within the biological sample. In particular, the sequences determined within the biological sample may be assigned a location within the biological sample based on the location

Preferably, the method is used to analyse genetic modifications in genetically modified cells.

Thus, by carrying out both, the amplification and the sequencing steps within the biological sample, the determined sequence of the amplified target sequence may be assigned to a location within the biological sample based on the location of the light emitted by the sequencing reaction within the biological sample.

### Short Description of the Figures

Hereinafter, specific embodiments are described referring to the drawing, wherein:
- Figure 1A: is a schematic view of a biological sample,
- Figure 1B: is a flow chart of a method for sequencing within the biological sample,
- Figure 2: is a schematic overview of the method compared to *in situ* LAMP,
- Figure 3: is a schematic overview of the different analytes that can be analysed using the method,
- Figure 4: schematically depicts LAMP-RT with universal cDNA synthesis and priming vs. sequence-specific priming,
- Figure 5: shows a schematic of the method for high-plex plasma proteomics,
- Figure 6: schematically illustrates how the method can be used for high-plex reading of barcoded affinity reagents and their respective targets, and
- Figure 7: provides a schematic overview of cell therapy quality control and therapy monitoring using the method.

### Detailed Description

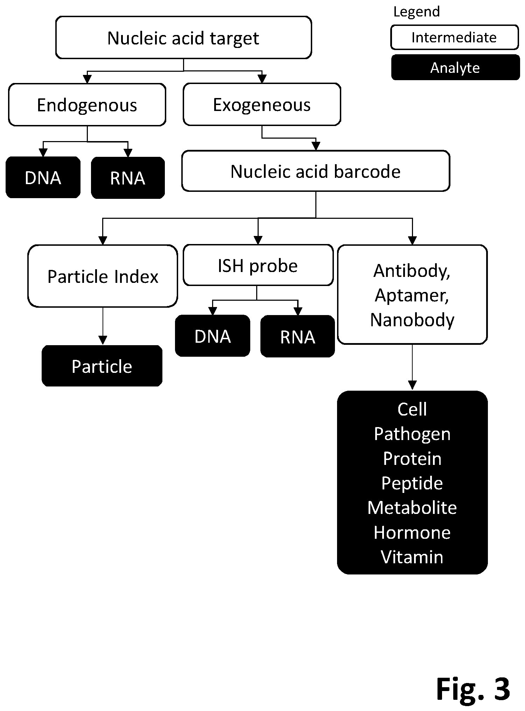

Figure 1A is a schematic view of a biological sample 100 and Figure 1B is a flow chart of a method for sequencing within a biological sample 100. The method starts in step S100. In step S102, a biological sample 100 is provided that comprises at least one target sequence 102 a, b, c, d. The biological sample may be a tissue section comprising a plurality of cells that may contain nucleic acids sequences such as mRNA 102b and DNA 102a (endogenous nucleic acid target) or may be an oligonucleotide barcode 104 labelling a ISH probe 102c, antibody 106, nanobody 108 or aptamer 110. An overview of different nucleic acid targets is provided in Figure 3.

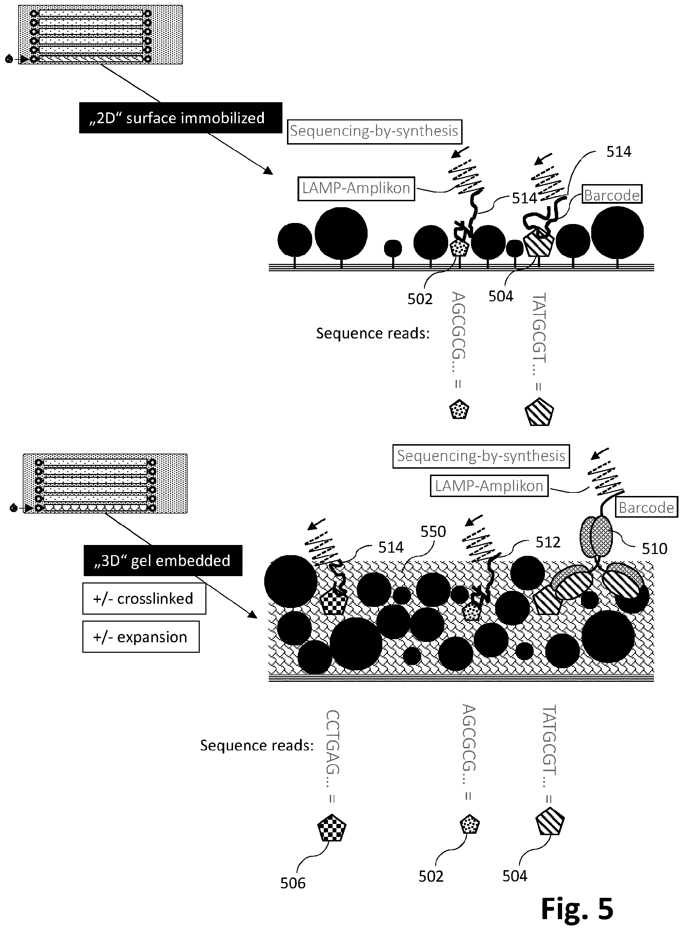

The target sequence 102a, b, c, d may be one of these nucleic acid sequences. The biological sample 100 may be a liquid sample or may be derived from a liquid sample or a solid sample. The liquid sample may be a lysate or a liquid biopsy, e.g. blood sample, plasma sample, serum, saliva, semen, lymph. The liquid sample may be further processed into a solid sample by fixing components, e.g. proteins on the surface of a slide or flow cell, or by embedding said components in a gel, e.g. a hydrogel, poly acryl amide (PAA), or similar. The former is referred to as "2D" surface immobilized the latter as "3D" gel embedded as shown in Figure 5. The biological sample may further a cell culture, 3D cell culture, organoid, tissuoid or spheroid sample, a cleared sample, a whole mount, or a tissue section for example.

In step S104, the target sequence is amplified within the biological sample with an isothermal amplification method. This generates at least one amplified target sequence. The amplified target sequence may be continuous and double stranded comprising a plurality of copies of the target sequence. Alternatively, several amplified target sequences may be generated that each comprise a plurality of copies of the respective target sequence.

The isothermal amplification method is preferably carried out at a low temperature compared to polymerase chain reactions, that utilised thermocycling with temperatures reaching up to 98° C. This limits any thermal degradation of the biological sample. For example, the isothermal amplification method is carried out at a constant temperature in a range from 20°C to 75°C. It is preferred, when the isothermal amplification method is carried out at a temperature in a range from 30 °C to 50 °C, in particular at 40 °C. This further reduces a build-up of fluorescent background in the biological sample.

The isothermal amplification method may initiate amplification by providing a set of primers comprising 4 to 6 primers. The primers cause the target sequence to loop back on itself. This provides free ends to initiate amplification of the target sequence, for example, by a strand displacing polymerase.

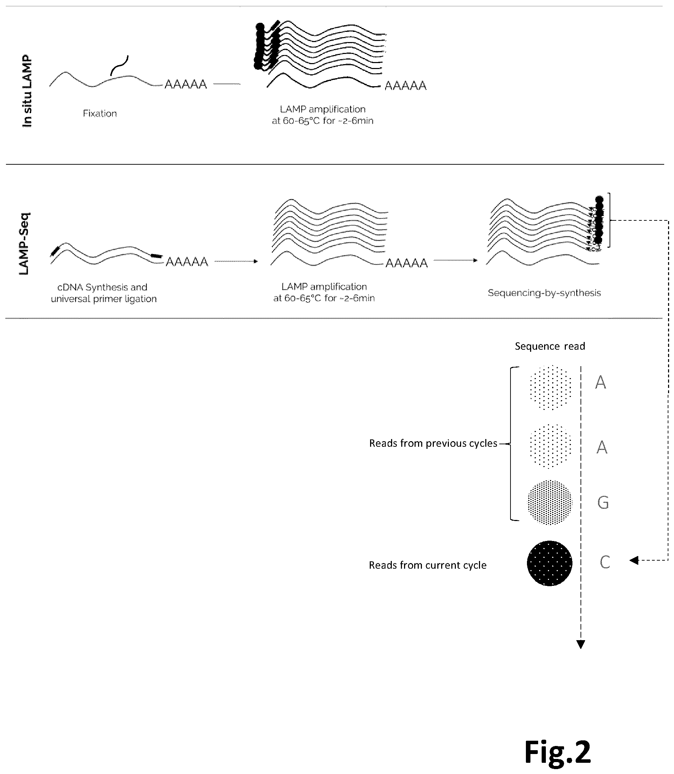

A particularly preferred isothermal amplification method is provided by Notomi et al, 2000, in particular, Fig. 1 therein and Podushkina et al., 2019, in particular, Fig. 1 therein. In Figure 2 reference is made to the method disclosed in Podushkina et al., 2019 as *"in situ* LAMP". In essence *"in situ* LAMP" renders the ISH signal much brighter, which aids in detection of lowly expressed mRNA. The present invention, likewise, leverages LAMP to perform amplification *in situ.* However, a different readout strategy is applied, which is *in situ* sequencing, i.e. reading at least a part of the nucleotide sequence using a sequencing-by-synthesis approach. The difference between the two is schematically shown in the lower part of Figure 2.

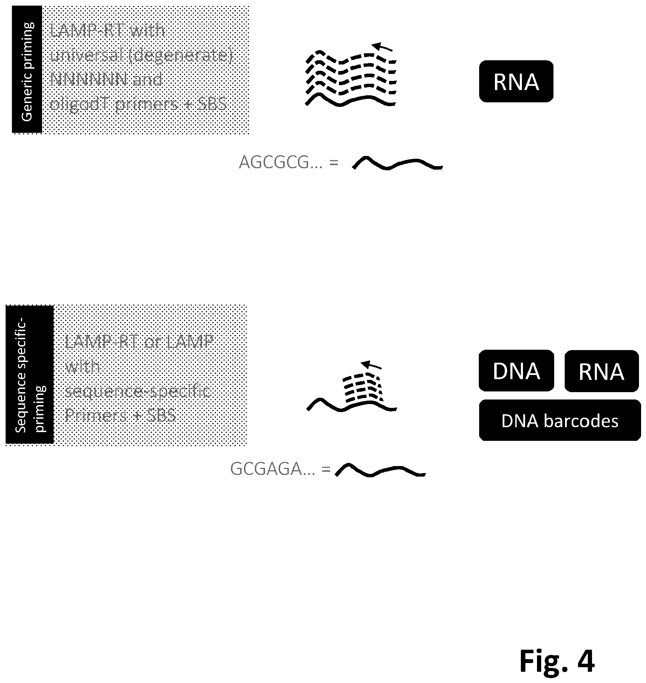

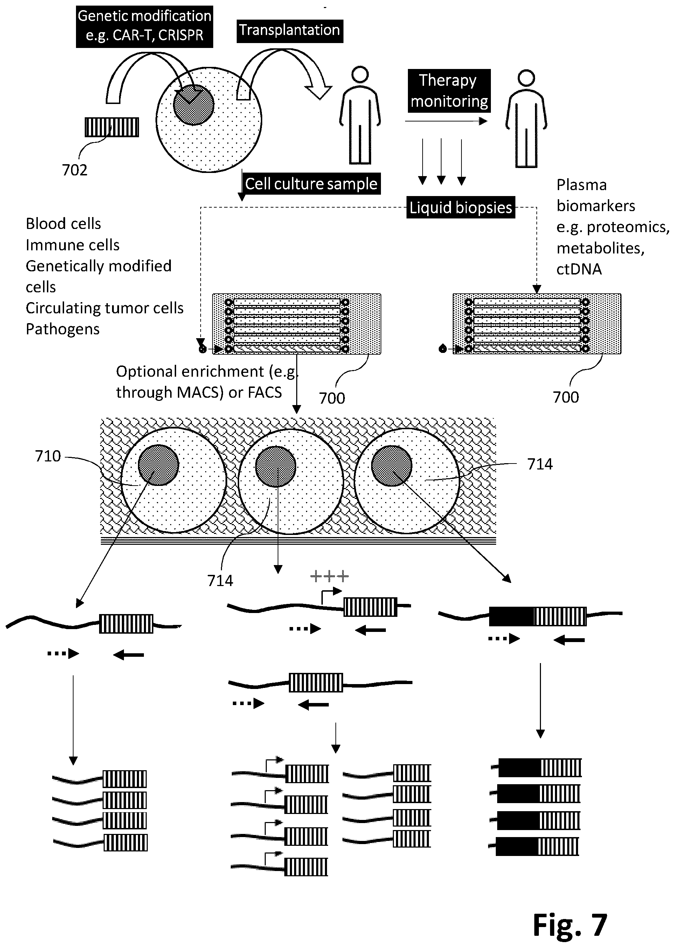

In case the target sequence is a mRNA the target sequence is initially transcribed by addition of a reverse transcriptase in the amplification step. This generates a cDNA target sequence that may be used to generate the amplified target sequence. It is important to point out that cDNA generation may be performed using sets of primers to amplify a certain mRNA or a certain set of mRNA ("sequence-specific priming"; Figure 4 bottom) or it may be performed using oligo-dT primers in combination with degenerate primers to amplify any mRNA, irrespective of which gene it is derived from ("generic priming"; Figure 4 top). Similarly, when the method is used to analyse the presence of an integration, site(s) of an integration, or copy number of an integration of gene therapy or cell therapy genetic modification a sequence-specific primer binding in the insert may be used in conjunction with a degenerate primer as depicted in Figure 7.

After addition of the set of primers, the amplification step is carried out for a period of time in a range from 0.5 min to 60 min. A range from 1 min to 15 min is particularly preferred. This results in an approximately 100- to 10000-fold increase in the number of copies of the target sequence. These copies of the target sequence may form the amplified target sequence. For an mRNA target sequence that is amplified to about 30 copies this is sufficient to generate a fluorescent spot corresponding to about 30 dye molecules that can be readout using a confocal microscope albeit with comparably low SNR. To enable fast and robust readout and base calling an amplification to about 1,000 copies may be particularly preferred.

Optionally, the generated amplified target sequence may be crosslinked. For example, the amplified target sequence may be attached to the biological sample or components thereof, such as proteins. Alternatively or additionally, a gel may be introduced into the biological sample. Further, at least one of the primers of the first set may comprise a chemical modification that enables its crosslinking, for example, to a component of the biological sample or a hydrogel or polyacrylamide gel that is introduced to the sample. This ensures that the amplified target sequence remains fixed in place within the biological sample and may be particularly useful, when the biological is expanded to increase the distance between spots, wherein a spot may correspond to a given nucleic acid target and its amplicon. Increasing the spot-to-spot distance may facilitate optical readout especially in densely labelled samples.

The method continues in step S104 with sequencing of the amplified target sequence within the biological sample. Generally, a sequencing by synthesis method may be used in this step. The sequencing of the amplified target sequence is preferably based on an optically detectable sequencing method. For example, the sequencing may comprise detecting light emitted from the biological sample. The light might be emitted by a nucleotide that is incorporated into a complementary strand during replication of the amplified target sequence within the biological sample, for example.

An example of such a method is pyrosequencing. Alternatively, the sequencing may comprise introducing labelled nucleotides into the biological sample, which are subsequently incorporated into a complementary strand during replication of the amplified target sequence within the biological sample.

The light emitted during sequencing of the amplified target sequence may be detected by means of an optical readout device, such as a microscope. This enables efficient in situ sequencing and spatially assigning the generated sequencing data within the biological sample. For example, during the sequencing step the biological sample may be imaged by means of the microscope and the detected emission light of the sequencing reaction allows determination of the sequence and its location as the source of the light within the biological sample. The method ends in step S106.

Thus, by carrying out both, the amplification and the sequencing steps within the biological sample, the determined sequences may be assigned a location within the biological sample which might be obtained by the optical readout. At the same time, the biological sample remains intact.

A second target sequence may be sequenced similarly to what is described above by providing a second set of primers specific to the second target sequence. This enables sequencing of a plurality of target sequences in parallel within the biological sample.

Figure 3 provides an overview of how the method for sequencing within a biological sample may be deployed to analyse different types of analytes (black boxes), like for example DNA, RNA, proteins, particles, cells, pathogens like bacteria and viruses, or small molecules, hormones, peptides, metabolites, hormones, vitamins. As a nucleic acid sequencing method, the method can be used to amplify and read nucleic acid targets in a biological sample. Such a nucleic acid target may be endogenous DNA (e.g. genetic loci, gene regulatory sequences, coding sequences, non-coding sequences, telomeres) or RNA (e.g. mRNA, rRNA). In this case, endogenous mRNA or genomic loci may not only be detected in a way that is compatible with high-plex (~1,000) or ultra-high plex (1,000 to 10,000 or 10,000 - 100,000 analytes) readout, but it is detected by actually reading the actual sequence of each individual target, which provides further layers of information in addition to mere analyte identification. For example, 500 mRNA molecules of gene X may be read (i.e. sequenced) in a cell using the method, and it may be found that there are 5 distinct splice isoforms a, b, c, d, e and that a certain fraction of the transcripts therefore contains an exon, which bears a mutation in ~50% of the corresponding transcripts, indicating heterozygosity with respect to the mutated allele. This sets direct reading of endogenous DNA or RNA apart from indirect detection of the same using barcoded ISH probes (indirect detection of the endogenous DNA or RNA target by sequencing a barcode of a ISH probe). In this case the method still provides numerous advantages over other high-plex RNA imaging methods, which are based on combinatorial encoding and fluorescent markers and generally require cyclical processes over many rounds to reach plexing levels in the range of 100s to around 1,000.

While the method lends itself well for a wide variety of assays, the most native application field is the *in situ* sequencing of mRNA, using LAMP-RT with generic priming of cDNA synthesis shown in Figure 4 (top), which provides:
- unbiased cDNA synthesis of transcripts and amplification for exploration of the transcriptome and gene expression profiling on the single cell level *in situ.*
- mRNA counts per gene per cell as an absolute measure of mRNA expression level (similar to what high-plex RNA imaging solutions that are based on combinatorial encoding and fluorescent in situ hybridization can achieve, albeit with much higher plexity and faster time-to-result)
- mRNA sequences that allow determination of splicing and detection and quantification of mutations or single nucleotide polymorphisms.

The method may further be used to profile certain sets of mRNA (i.e. from a certain set of genes) using sequence-specific primers shown in Figure 4 (bottom) that may amplify the entire open reading frame or only a fraction necessary to determine the identity of the analyte. Similarly, sequence-specific priming may be used on exogenous nucleic acid targets, such as DNA oligonucleotide barcodes that may be attached to antibodies, aptamers, or ISH probes. As stated before, it is a particular advantage of the method that the detection and analysis by sequencing of endogenous DNA and RNA can be combined seamlessly with the sequencing of DNA barcodes on affinity reagents that mark protein biomarkers for example. While there may be a situation, in which crowding of proteins may become a limiting factor for an optical readout, this is not always the case and may be mitigated by expanding the sample or in the case of liquid samples can be resolved by choosing an appropriate assay design with respect to sample carrier design, embedding and expansion of the sample.

Furthermore, the method may be used to read barcodes on any affinity reagent including antibodies, nanobodies, or aptamers *in situ.* In this sense it is important to note that a biological sample may also be a liquid biopsy, like for example a plasma sample, that may for example be immobilised in a gel. A corresponding example is depicted schematically in Figure 5. In this case, high plex analysis of target analytes 502, 504, 506 may be performed using large libraries or barcoded antibodies 510 or aptamers 512 or aptamer-derivatives 514 like SOMAmers or aptabodies. The advantage of sequencing barcodes *in situ* in contrast to cutting barcodes off and sequencing them in bulk on a next generation sequencing readout is that the individual binding event is detected and visualized, which allows the correlation of multiple binding events to one target, which may occur simultaneously or in a temporally sequential manner. Using multiple affinity reagents, to detect the same analyte may be used to increase the specificity of an assay, which is particularly important, when multi-, high-, or ultra-high plex analysis is being performed as such assays are generally severely limited by the cross-reactivity of affinity reagents to OFF-targets.

The particular advantage of the use of this method for proteomics is that proteomic samples are prepared from liquid samples and that analytes can be immobilized on surfaces or in gels 550 (e.g. hydrogels) and that they can be cross-linked and that the distance between analytes can be easily tuned in various ways (e.g. by dilution, increasing/decreasing surface or volume, expansion) to the ideal point with respect to the resolving power of the readout. This is schematically depicted in Figure 5 which shows how immobilizing proteins of a plasma sample on a surface of a flow cell channel (Fig, 5, top) or in a hydrogel (Fig. 5, bottom) can be used to this end. In the Figure 5 "mass proteins" of the plasma such as albumin, transferrin, alpha-2-macroglobulin or fibronecting also referred to as "classical plasma proteins" are represented as black circles. Usually these proteins are of secondary interest, whereas the highest interest lies in analytes that have medium to extremely low abundance - the dynamic range between albumin (mg/ml) and the low abundance proteins ( µg/ml, ng/ml, pg/ml) being about 10¹⁰-10¹². Such low abundance proteins, which are frequently of great interest as they carry most of the predictive value in the sense of patient stratification for clinical studies or of companion diagnostics that help in monitoring and guiding therapy in the clinic are tissue leakage products and other low abundance proteins such for example VEGF, colony stimulating factor-1 and other cytokines or interleukins.

In this way the method for sequencing within a biological sample allows very simple fluorescence microscopes to be used in combination with expanded hydrogel embedded preferably cross-linked samples to perform high-plex or ultra-high plex proteomic analysis without necessitating the use of combinatorial fluorescent labels or complicated cyclical encoding and decoding processes. Sequencing chemistries that are being used for next generation sequencing such as 1-color, 2-color, 4-color chemistries may be used. Likewise, immobilization on a surface of a flow cell may be used preferably with a TIRF (total internal reflection fluorescence microscopy) microscope or a widefield fluorescence microscope and the method for sequencing within a biological sample, to perform high-plex or ultra-high plex analysis of plasma proteomic samples. This may also facilitate the concomitant analysis of multiple classes of biomarkers by combining the sequencing of endogenous DNA, RNA biomarkers with the sequencing of nucleic acid barcoded antibodies or aptamers for other analyte classes like proteins, hormones, metabolites, etc. In this way the method may be used to build an end-to-end application solution for plasma analysis in the context of precision medicine that allows the interrogation of genetic loci (e.g. for mutations, SNPs, copy number variations), mRNA expression and splicing, as well as protein, hormone, metabolite biomarkers using one unified protocol, chemistry and device for the readout.

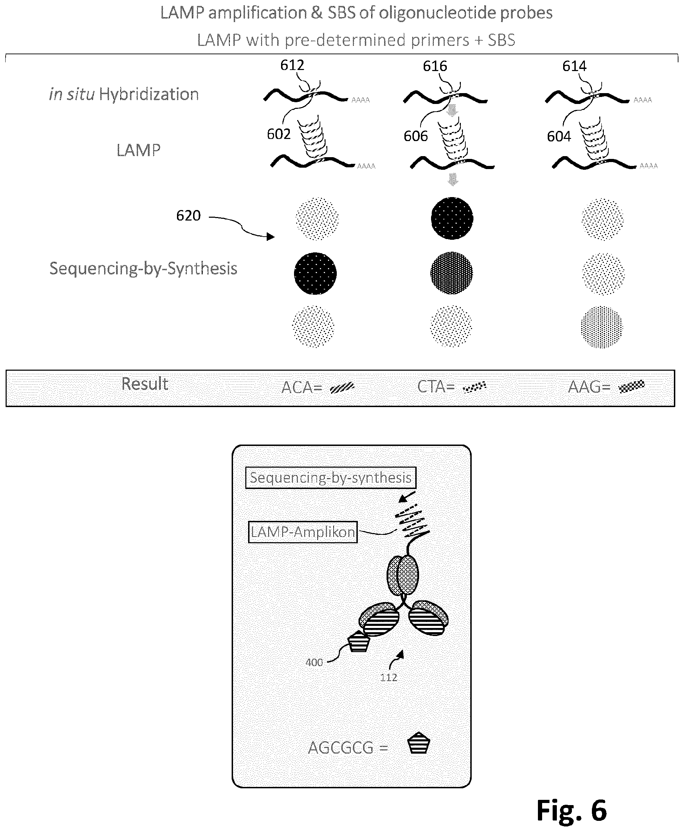

The use of the method with barcoded ISH probes or barcoded antibodies is further schematically depicted in Figure 6. Two mRNA targets 602, 604 and a DNA target 606 are bound in a first step by a respective *in situ* hybridization (ISH) probe with a complementary sequence-specific to the target (patterned rectangles; 612, 614, 616). The overhangs of the sequence indicated by the U-shape is used for priming for the LAMP reaction, which allows robust and fast amplification resemble by the stacking of U-shaped ISH probes (in reality these may be concatemers or groups of concatemers). During the sequencing-by-synthesis (SBS) step each RNA or DNA spot is optically readout and a sequence of colours 620 is generated and then translated to a sequence read "result", which identifies the target. A comparably short sequence stretch may already be sufficient to identify the target 602, 604, 606. While this method is not fully exploratory, as targets have to be known upfront, it provides the advantage that the same set of LAMP primers can be used to amplify an entire group of barcodes. Such a group may consist of barcodes on ISH probes marking RNA and/or DNA and/or barcodes on other affinity reagents that mark proteins for example. Again, this renders multi-analyte class high plex analysis particular efficient and easy as well as compatible with a comparably simple fluorescent microscopic readout.

In a further embodiment of the present invention shown in Figure 7 the method is used to perform quality control in cell line development workflows like for example, for cell therapy products, wherein an autologous or heterologous population of cells is genetically modified to include a genetic insert cassette 702, that is integrated into the genome. In such cases it is of great interest to determine the effectiveness of genetic modification. To this end cell culture sample may be taken and analysed for the integration. Likewise, following to transplantation, i.e. during the course of a cell therapy it is important to monitor the population of genetically modified cells as well as the patient response to the treatment. Blood borne biomarkers play a key role at this point. As detailed in Figure 5, the method for sequencing within a biological sample may be used to perform plasma proteomic analysis of patient samples to assess key biomarkers and stratify patient populations. Furthermore, it may be used as depicted in Figure 7 and cells from a blood sample may be immobilized in a lane of the chip 700. The chip is adapted to be optically read out by a light microscope and comprises six lanes in which the cells can be immobilized. Now a sequence-specific primer configured to hybridize to a sequence stretch inside the genetic insert cassette is used alongside a degenerate primer, which may bind outside of the insert at random positions in the genome. In this way, a genetic insert cassette can be at least partially amplified alongside some fused genomic sequence, which allows the location of the insert and detection of multiple insertions. In the schematic example the cell 710 on the left has an intergenic integration, the cell 712 in the middle has two integration one of which is right behind a strong promoter, the other intergenic, the cell 714 on the right has an integration that fuses the insert to an exon of a gene. This illustrates how the method for sequencing within a biological sample may be used to perform fine-grained analysis of cells on the genetic level, while keeping them intact which allows phenotypic characterization of the same cells using microscopic assays. In analogous way, large numbers of cells either from a cell culture, liquid biopsy, or tissue section sample may be analysed for key aspects of their genetic makeup, like for example certain genes or gene sets may be probed or specific regions, and this information may be correlated to a variety of imaging-based phenotypic assays (e.g. cell morphology, marker expression, etc.). In this way the method for sequencing within a biological sample enables high-throughput genotype to phenotype correlation for large populations of cells on the single cell level.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 102a: DNA nucleic acid target
- 102b: mRNA nucleic acid target
- 104: oligonucleotide barcode nucleic acid target
- 106: antibody
- 108: nanobody
- 110: aptamer
- 602: mRNA target A
- 604: mRNA target B
- 606: DNA target
- 612: complementary sequence-specific to the target 602
- 614: complementary sequence-specific to the target 606
- 616: complementary sequence-specific to the target 604
- 620: sequence of colours
- 502, 504, 506: non-nucleic acid target analyte (e.g. protein)
- 510: antibody
- 512: aptamer against 502
- 514: aptamer-derivative (e.g. SOMAmer, aptabody)
- 550: (hydro-)gel
- 700: (microfluidic-)chip
- 702: genetic insert cassette
- 710: cell on the left
- 712: cell in the middle
- 714: cell on the right

## Claims

1. A method for sequencing within a biological sample, comprising the following steps:
providing the biological sample comprising at least one target sequence,
amplifying within the biological sample the target sequence with an isothermal amplification method in order to generate at least one amplified target sequence, and
sequencing within the biological sample the amplified target sequence.

2. The method according to claim 1, wherein the isothermal amplification method comprises introducing a set of primers into the biological sample, the primers of the set of primers configured to hybridise to the target sequence.

3. The method according to claim 2, wherein the primers of the set of primers are configured to generate hairpin loops at ends of the target sequence.

4. The method according to any one of the claims 2 or 3, wherein the set of primers comprises at least a first primer with a first region of the first primer being configured to hybridise to at least the target sequence towards a 3' end of the target sequence, and a second region of the first primer being configured to hybridise to the amplified target sequence downstream of the first region of the first primer.

5. The method according to any one of the claims 2 to 4, wherein the set of primers comprises at least a second primer with a first region of the second primer being configured to hybridise to the amplified target sequence towards a 3' end of the amplified target sequence, and a second region of the second primer being configured to hybridise to the amplified target sequence downstream of the first region of the second primer.

6. The method according to any one of the preceding claims, wherein the amplified target sequence is a continuous sequence comprising multiple copies of the target sequence.

7. The method according to any one of the preceding claims, wherein the isothermal amplification method comprises introducing a strand displacing polymerase into the biological sample.

8. The method according to claim 7, wherein the strand displaying polymerase is a Phi29 polymerase, a Bacillus subtilis DNA polymerase I, a T7 DNA polymerase, or a T4 DNA polymerase.

9. The method according to any one of the preceding claims, wherein the biological sample is permeabilised and/or expanded.

10. The method according to any one of the preceding claims, wherein the step of amplifying the target sequence is carried out at a substantially constant temperature in a range between 20°C to 75 °C.

11. The method according to one of the preceding claims, wherein the step of amplifying the target sequence is carried out for 0.5 min to 60 min.

12. The method according to any one of the preceding claims, wherein after the step of amplifying the target sequence, the amplified target sequence is crosslinked.

13. The method according to any one of the preceding claims, wherein the biological sample comprises a second target sequence, and wherein the step of amplifying the target sequence comprises introducing a second set of primers in order to amplify the second target sequence, wherein the primers of the second set are specific to the second target sequence.

14. The method according to one of the preceding claims, wherein the step of sequencing the amplified target sequence comprises introducing labelled nucleotides into the biological sample.

15. The method according to one of the preceding claims, wherein the step of sequencing the amplified target sequence comprises detecting light emitted when a nucleotide is incorporated into a complementary strand during replication of the amplified target sequence.

16. The method according to one of the preceding claims 14 to 15, wherein the step of sequencing the amplified target sequence comprises detecting light emitted from the biological sample by means of an optical readout device.

17. The method according to one of the preceding claims, wherein the biological sample is derived from a liquid sample by fixing at least some of the analytes contained in said liquid sample substantially in place.

18. The method according to one of the preceding claims, wherein the biological sample is a synthetic sample by mixing components of a biological sample with a hydrogel.

19. The method according to one of the preceding claims, wherein the biological sample comprises exogenously applied or introduced nucleic acid target sequences.
